# EUROPEAN PATENT APPLICATION

(11) **EP 2 628 748 A1**
(43) Date of publication of application: **21.08.2013**
(21) Application number: 12155368.9
(22) Date of filing: 14.02.2012
(51) Int. Cl.: C07K 14/515, C07K 14/435, C12N 5/00, A61K 48/00

(54) **Angiostatin chimeras and uses thereof**

(71) Applicant: Szilak Laboratories Bioinformatics & Molecule-Design Ltd., 6723 Szeged (HU)
(72) Inventor: Szilák, László, 6723 Szeged (HU); Tímár, József, 1112 Budapest (HU); Keller-Pintér, Anikó, 6640 Csongrád (HU); Letoha, Tamás, 6725 Szeged (HU); Katona, Róbert, 6754 Újszentiván (HU)
(74) Representative: Lengyel, Zsolt

(57) **Abstract**

The invention relates to a chimeric fusion protein comprising angiostatin releasable therefrom by cleaving a protease-sensitive site comprised therein, wherein the fusion protein comprises a protease. Further the invention provides related nucleic acids, vectors and host cells, as well as uses as methods involving said chimeric protein.

## Description

The invention relates to a chimeric fusion protein comprising angiostatin releasable therefrom by cleaving a protease-sensitive site comprised therein, wherein the fusion protein comprises a protease. Further the invention provides related nucleic acids, vectors and host cells, as well as uses as methods involving said chimeric protein.

The endothelial cell proliferation is inhibited by proteolytic fragments of plasminogen, or more specifically by the kringle structures (1-5) of plasminogen. The term "angiostatin" corresponds to the functional domains of plasminogen fragments compressing various members of the five kringle structures, which, thereby is able to block endothelial cell proliferation. According to the classical terminology, the term angiostatin meant strictly the 1-3 kringles, however the state of the art taught us that 1-4 kringles, 4th and 5th kringle alone had anti-angiogenic effect (Liu et al., 2000). Angiostatin was discovered and proven as an agent which blocks vessel neoformation and growth in both primitive and metastatic tumors via inhibition of endothelial cell proliferation (O'Reilly et al., 1994, Sim et al., 1997; Cao et al., 1998; Whal et al., 2004, US5837682). Angiogenesis is the process in which endothelial cells in the lumen of blood vessels protrude through the basement membrane and migrate through the eroded basement membrane. The migrating cells form a "sprout" off the parent blood vessel, where the endothelial cells undergo mitosis and proliferate. The endothelial sprouts merge with each other to form capillary loops, creating the new blood vessel.

Hereafter the term "angiogenesis" means the generation of new blood vessels into a tissue or organ.

Despite promising results in mice, it has not been possible to produce clinical grade soluble, active angiostatin in commercial quantities using *E. coli,* baculoviral, yeast, and mammalian expression systems. Expression in prokaryotic cells, e.g. in *E. coli,* yielded insoluble protein aggregates of undefined composition, which could not be injected into humans. Other production methods, such as baculovirus and mammalian expression systems, yielded very low levels of the recombinant proteins (Moser et al., 2002; Wahlet al., 2004; and 2005).

Other patents disclose angiostatin sequence variants and methods to express, isolate, purify and synthetically produce it, see e.g. US patents No. 5861372; 5639725; 5792845; 5885795; 5854205; 5854221; 6024688, WO9929878, WO9916889 and others e.g. CN101671675, KR20000073337.

Because endothelial cells are genetically stable, it is possible that angiogenesis inhibitor therapies may result in less drug resistance. Studies indicate that drug resistance did not develop in mice exposed to prolonged anti-angiogenic therapy using endostatin. Furthermore, repeated cycles of endostatin treatment in mice resulted in prolonged tumor dormancy and no recurrence of tumors following discontinuation of therapy. In angiostatin-treated animals, neither toxic effects nor development of resistance have been observed (Boehm et al. 1997).

It can be seen from the above discussion of the state of the art that the currently available methodologies are in need of substantial improvements to provide better tools for preventing angiogenesis and fighting tumors. The present inventors accomplish this goal by providing an efficient, stable and affordable way to introduce angiostatin and ensure that it is available at suitable levels for allowing effective inhibition of vascularization of newly formed tumors and metastases.

Accordingly, the present invention provides a chimeric fusion protein comprising angiostatin releasable therefrom by cleaving a protease-sensitive site comprised therein, wherein the fusion protein comprises a protease.

In another embodiment, the invention provides a fusion protein, wherein said protease-sensitive site is cleavable by the protease comprised in the fusion protein.

In another embodiment, the invention provides a fusion protein, wherein the amino acid sequences of angiostatin and the protease is separated by a linker sequence consisting of 3 to 15 amino acids, preferably comprising the protease sensitive site. In more specific embodiments the linker can be of any length between 3 and 15 amino acids.

In another embodiment, the invention provides a fusion protein, wherein the protease is elastase.

In another embodiment, the invention provides a fusion protein, wherein the C terminus of the angiostatin is linked to the N terminus of the protease. In another embodiment, the invention provides a fusion protein, wherein the N terminus of the angiostatin is linked to the C terminus of the protease.

In another embodiment, the invention provides a fusion protein, further comprising at least one targeting sequence, preferably for transporting the fusion protein to the extracellular space or to a specific type of cell or tissue.

In another embodiment, the invention provides a nucleic acid encoding said fusion protein.

In another embodiment, the invention provides an expression vector comprising said nucleic acid.

In another embodiment, the invention provides an expression vector, comprising regulatory sequences allowing constitutive or inducible expression.

In another embodiment, the invention provides a host cell comprising said expression vector.

In another embodiment, the invention provides a host cell, wherein the cell is a stem cell, most preferably mesenchymal stem cell, or other isolated cell, which can be grown to clinically effective cell numbers.

In another aspect, the invention provides a chimeric fusion protein, comprising angiostatin releasable therefrom by cleaving a protease-sensitive site comprised therein, or a nucleic acid encoding said chimeric fusion protein, or a vector or host cell comprising said nucleic acid, for use in inhibiting angiogenesis, preferably in cancer or tumor.

In another embodiment, the invention provides a method for the inhibition of angiogenesis, comprising *in situ* release of angiostatin from the fusion protein according to the invention, or from the protein encoded by the nucleic acid according to the invention or the expression vector according to the invention, or from the host cell according to the invention.

In another embodiment, the invention provides a method according, wherein the angiostatin is released by autocatalysis, upon induction or constitutively.

### Detailed description

In accordance with the present invention, compositions and methods are provided that are effective for modulating angiogenesis, and inhibiting unwanted angiogenesis, especially angiogenesis related to tumor growth. The present invention provides a chimeric gene for a protein that can be produced *in situ* in the patients, which comprises two functional units: a protease and the kringles of plasminogen named "angiostatin", defined by its ability to overcome the angiogenic activity. This chimera gene upon expression in proper cells within the host will produce the fusion proteins, which is thereafter capable of releasing the active angiostatin constitutively or inducibly by proteolytic cleavage. Therefore the invention provides the *in situ* release of active angiostatin.

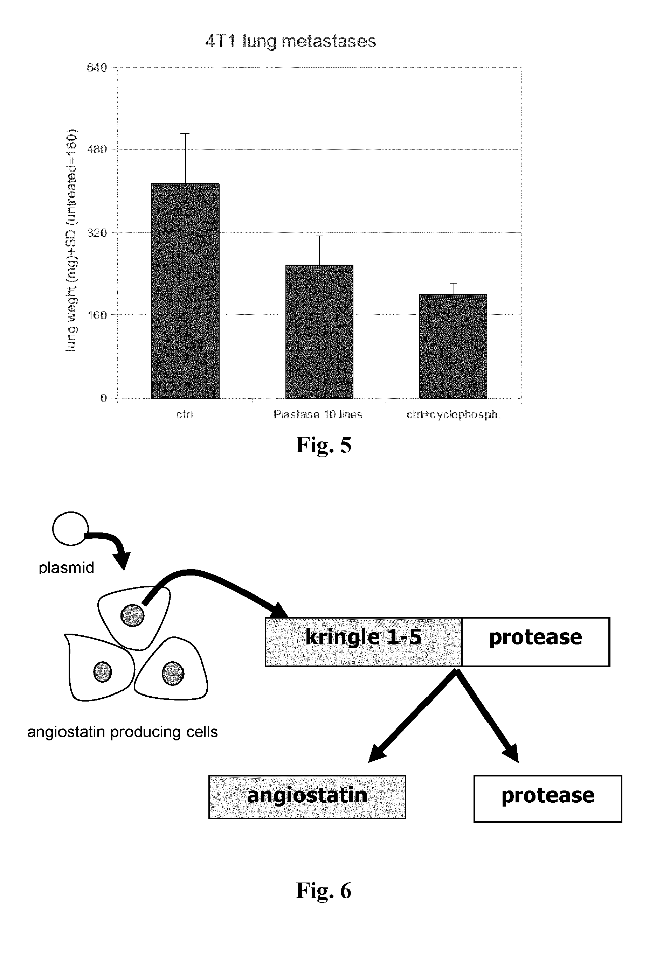

In a first embodiment of the present invention, a chimeric fusion protein is provided that comprises angiostatin which is releasable by cleaving a protease-sensitive linker. The fusion partner itself is a protease. It can be safely assumed that the work by Cao et al. (1998) can be considered as the closest prior art. They discloses the cloning of 4 kringles of plasminogen as angiostatin and implant the generated cells into mice to study the anti cancer effects. However the release of the active angiostatin is enigmatic. It is clear for the person skilled in the art that the present invention differs on several points from this prior teaching, most notably by including the full structure of the angiostatin that is the five kringles of plasminogen in a chimeric fusion protein, which provides a fully active protein upon release by proteolytic cleavage. In certain specific embodiments, this proteolytic cleavage is predominantly autocatalytic, that is said protease-sensitive linker is cleavable by the chimeric protease, however the release of the active angiostatin can be inducible in a proper construct designed for inducible cleavage. Upon entering an environment where the conditions are suitable for the cleavage, the protease-sensitive linker is cut to release the active angiostatin. This general concept is shown on the flow chart presented in Fig. 6, schematically depicting the expression and action of the present angiostatin chimera *in vivo.* Stem cells or any other cells that can be introduced to human or somatic human cells are transfected with angiostatin-chimera. Upon introduction of the transfected cells back to host they start producing the chimera, which subsequently cleaved to release the active angiostatin so blocking the migration and the proliferation of endothelial cells. According to the present invention the protease part of the chimera can be any protease which has the capability of releasing angiostatin by cleaving at the proper amino acid sequence of said chimera. In a preferred embodiment, the protease is preferably elastase. In further preferred embodiments, the protease-sensitive linker of the chimera is cleavable by elastase, therefore is capable of releasing the active angiostatin. Further, linkers can be designed with specific cleavage sites, which would allow an even more controlled release of angiostatin from the chimera. When the cleavage site is for an enzyme which is not naturally occurring at the targeted site, the cutting enzyme may be provided in the form of another construct co-transfected into the same cell or alternatively into different cells, introduced at the same time or separately.

The gene of the fusion protein can be constructed with a fusion of the coding region of kringles and a protease following a secretion signal, which is able to orient the chimeras to be exported. According to the present invention there is no need for exobiotic expression and purification of angiostatin, because the angiostatin would be released *in situ, in vivo.*

The cells suitable to deliver the gene and express the chimera *in situ* according the present invention can be any cells from the host, which can be stably transfected with the chimera. The transfected cell then can be introduced back into the host expressing the chimera persistently. The cells are preferably stem cells, most preferably mesenchymal stem cells or isolated other cells, which can be grown to the clinical effective cell number, which characterized by clinically to satisfy the former criteria.

The expression of the chimera could be regulated by constitutive or inducible promoters, which can be tissue specific or not tissue specific. The person skilled in the art will readily be able to select the appropriate regulatory sequences which are useful to create the appropriate delivery system for the chimera of the invention. It is clear that the construction of the delivery vector and the choice of cells to deliver the chimera to the tumor site to be treated should be designed to allow for their full functionality.

The fusion protein according to the invention additionally may contain further elements, for example at least one targeting sequence to allow the specific delivery of the chimera to specific target sites. Target sequences are generally known for the person skilled in the art, it may be for example a sequence for transporting the fusion protein to the extracellular space, most preferably to the blood stream.

Suitable vectors carrying the chimera according to the present invention are widely known and commercially available, however in special cases a proper promoter usage would be needed. The person skilled in the art can develop and use the proper systems. The chimera of the present invention can be inserted into any of the appropriate vectors by standard techniques. Viral vector systems are also available for the person skilled in the art.

The present invention also provides host cell comprising the nucleic acid encoding the chimera of the invention or the expression vector comprising thereof. Suitable host cells are known for the person skilled in the art. As non-limiting examples, the person skilled in the art can use somatic cells e.g. epithelial cells of lung or stomach.

In specific embodiments, the host cell is a stem cell, most preferably mesenchymal stem cell, which can migrate directly to the cancerous foci, therefore the expression of the anti-angiogenic agent would happen in the cancerous foci. The term "stem cell" may include embryonic stem cells, provided that their use is otherwise acceptable. On the other hand, the person skilled in the art will be able to select any type of cells that can be grown to clinically effective cell numbers, and are suitable to be introduced into the patients to be treated.

The present invention also directed to a method for the inhibition of angiogenesis, comprising *in situ* release of angiostatin from the fusion protein according to the invention, or from the protein encoded by the nucleic acid according to the invention, or from the host cell according to the invention.

In a specific embodiment, the method according to the invention is for treating a patient suffering from cancer or a tumor. In the context of the present invention, treatment includes any type of administration of the chimera according to the invention to a subject where the purpose is to cure, heal, alleviate, relieve, alter, remedy, ameliorate, improve, or affect a tumor, any associated symptoms of said tumor, or the predisposition toward development of said tumor.

The type of said cancer or tumor is not limiting, but the method of the invention is especially suitable for the treatment of metastatic stage.

Treatment and prevention of tumor metastases forms a specifically advantageous embodiment of the present invention.

To effect the delivery of the chimera of the invention, the construct comprising thereof, or the recombinant host cell comprising thereof must be introduced into the organism to be treated. Such delivery techniques are readily available in the art for introducing nucleic acids, vectors and cells into living organisms.

In a further aspect, the chimera of the invention, the construct comprising thereof, or the recombinant host cell comprising thereof is used in a method for the treatment of the tumor as defined above. In that respect, a method is encompassed by the present invention as long as it is not a method for treatment of the human or animal body by surgery or therapy and/or a diagnostic method practised on the human or animal body. The person skilled in the art will be readily able to determine if the method falls under the scope of this exception.

### Description of the figures

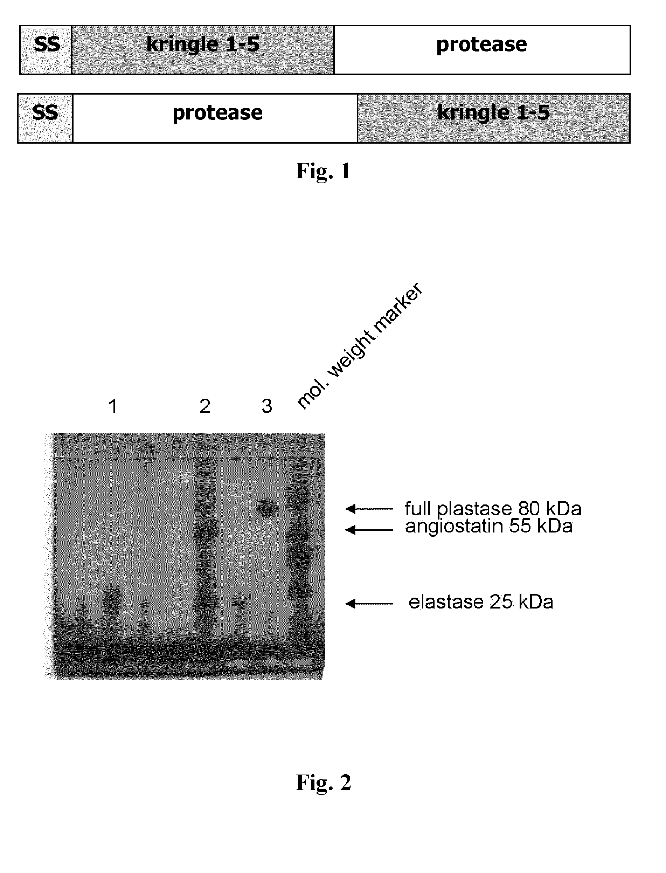

**Fig. 1****.** Possible structures for the angiostatin producing chimeras. SS: signal sequence. The protease can be positioned upstream and downstream of kringles.
**Fig. 2****.** Purification and autocatalysis of the chimeric protein. Purified elastase (lane 1) and full size angiostatin-chimera (lanes 2, 3) were loaded and electrophoretically separated on SDS-PAGE. The sample loaded in the 3rd lane was processed in the presence of protease inhibitor prior to electrophoresis. Lane 2 shows the proteolytic fragments of the angiostatin-chimera. This sample was purified, and handled without protease inhibitor.

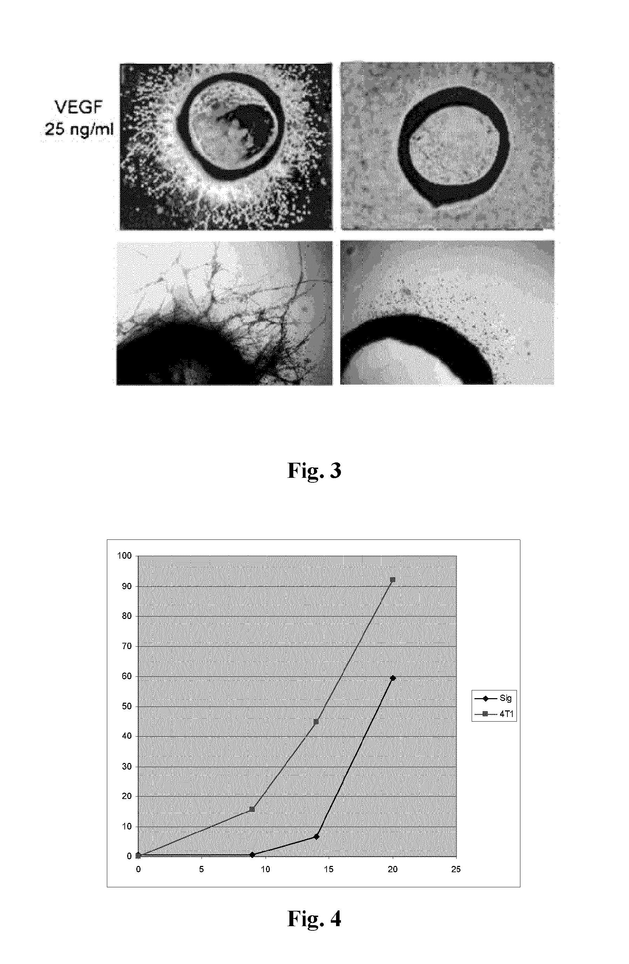

**Fig. 3****.** Angiostatin inhibits capillary formation in VEGF-induced aorta assay. Thoracic aortas from 4-6 week old rats were excised, and sliced for rings approximately 1 mm in length. The aortic rings were embedded in Matrigel, and covered by tissue culture media. Other cell types expressing anti-angiogenic factor(s) were co-cultured in cell culture inserts, which were immerged into the media. Neovessel outgrowth, which sprouted from the cut ends was monitored using phase microscopy for 5-6 days. On the left side panels the wild type cells did not block the vessel sprouting, however the transfected cells expressing angiostatin inhibited the neovessel outgrowth, shown in the right side panels.
**Fig. 4****.** Rate of tumor growth in diameter. The diameter of the tumors were measured and plotted in the average of 5 - 5 different mice introduced wild type 4T1 tumor cells (open square) and angiostatin-chimera transformed 4T1 (black diamond). After two weeks one of the wild type 4T infected mice died and the meantime two mice bearing angiostatin-chimera expressing 4T1 were sacrificed and they showed no detectable tumor.
**Fig. 5****.** Weight of metastatic tumors induced by wild type, transfected 4T1 cells. In the *in vivo* experiments, 2-3 months old BALB/c female mice were injected with 100 000 tumor cells suspended in 100 µl PBS. 10 mice were infected with the original 4T1 cells used as control, while the experimental group (10 mice) was injected with the transfected lines. We compared the effect of angiostatin expressing constructs to the potent drug cyclophosphamide. The length and width of the primary tumors were measured using digital calipers on days indicated. At the end point mice were over-anesthetized, then the internal organs were checked for the presence of macroscopic metastases, then lungs were removed and the weight was measured.
**Fig. 6****.** Flow chart of the mechanism of the expression of angiostatin *in vivo.* Stem cells or any that can be introduced to human are transfected with angiostatin-chimera. Upon introduction of the transfected cells back to host they start producing the chimera, which subsequently cleaved to release the active angiostatin so blocking the migration and the proliferation of endothelial cells.

### Example 1: Production of cells containing chimeras

Using state of the art techniques, angiostatin elastase chimeras were produced. The sequence shown in SEQ ID NO.: 1 represents an example for a possible arrangement of angiostatin - elastase chimera, wherein the angiostatin is N-terminally positioned of elastase.

The plasmids were introduced into the 4T1 cells with commercially available kits, standard techniques commonly available to a person skilled in the art of molecular biology.

### Example 2: Stability of the chimeras and release of angiostatin

Angiostatin-protease chimera constructs (Fig.1) were transfected to different cell lines and the medium was purified through poly-lysine column, and analyzed for angiostatin. We got the full size chimera (80 kDa) if the isolation happened in the presence of serine protease inhibitor mix, otherwise we could detect only the angiostatin and the protease itself (Fig. 2).

### Example 3: In vitro proof for release and action of angiostatin

To test whether the constructs work, endothelial cell line was propagated in hanging culture in a transwell insert about a millimeter off the bottom of the well, where the angiostatin-chimera expressing cell lines were propagated. This hanging design provides the same condition for both cultures, because the trans-well insert allow access to the lower compartment without letting the two cell cultures mix. In that design the endothelial cells stopped proliferating in the upper, hanging dish, when angiostatin-chimera expressing cell lines were propagated at the bottom.

### Example 4: Aorta test

Aorta-test was carried out for further investigation. The aortic ring assay is an *in vitro* angiogenesis model that is based on organ culture. In this assay, angiogenic vessels grow from a segment of the aorta. Briefly, thoracic aortas from 4-6 week old rats were excised, the fat layer and adventitia were removed, and rings approximately 1 mm in length were prepared. Individual rings were then embedded in Matrigel (Sigma), cast inside individual wells of a 48-well plate. The solid domes are covered with endothelial-SFM (ESFM) basal growth medium (Gibco). Angiogenic factors e.g. VEGF (25 ng/ml) was directly added to the rings. Other cell types expressing anti-angiogenic factor(s) were co-cultured in cell culture inserts, which are permeable devices hanging in the media of wells. Media was changed for fresh one after 3 days. Sprouting was observed by inspection under a stereomicroscope over a period of 5-6 days. Due to the large variation caused by the irregularities in the aortic segments, experimentation was executed in triplicates. Neovessel outgrowth, which sprouted from the cut ends was quantitated throughout the experiment and imaged using phase microscopy. Supernatants were collected for measurement of relevant anti-angiogenic factors. To stop the assay Diff Quick Fix Solution II was added after the ESFM media removed.

Fig 3 shows that the endothelial cells did not proliferate, when angiostatin-chimera bearing cells were cultured prior to layering the matrigel embedded aorta slices.

### Example 5: In vivo tests of the chimera

### In vivo murine tests

In one set of *in vivo* tests the 4T1 mouse mammary tumor virus (MMTV)-induced mammary carcinoma cells were used, inducing a highly metastatic, poorly immungenic BALB/c mouse tumor. The 4T1 tumor cell line was obtained from ATCC. Prior to the infection of mice tumor cells were prolongated in RPMI tissue culture medium in the presence of 10% FCS. The cells were transfected with the Angiostatin expressing constructs using the Lip.LTX transfection reagent (Invitrogen) then stable cell lines were generated. Ten independent G418 resistant clones were established and used in the further experiments.

5 - 5 different mice were injected with wild type (wt) 4T1 tumor cells, initiating breast cancer (Fig. 4, squares) and angiostatin-chimera transformed 4T1 (Fig. 4, diamonds). After two weeks one of the wild type 4T1 infected mice died and at meantime two mice bearing angiostatin-chimera expressing 4T1 were sacrificed for checking the tumor growth. The two mice showed no detectable tumor in the first two weeks. Later on tumor growth were detected but with a much slower rate that in the case of wt-4T 1. The diameter of the tumors were measured and plotted in Fig. 4.

In other *in vivo* experiments, 2-3 months old BALB/c female mice were injected with 100 000 tumor cells suspended in 100 µl PBS. Control mice received the original 4T1 cell line, while the experimental group received the transfected lines. Each group contained 10 mice. The length and width of the primary tumors were measured by caliper. At the end point mice were over-anesthetized, then the internal organs were checked for the presence of macroscopic metastases, then lungs were removed and the lung weight was measured.

Tumor growth was compared in the cases of the non-treated 4T 1, the angiostatin producing 4T1, and the 4T1 infected and cyclophosphamide treated mice. The cyclophosphamide is known as a potent drug, specially used for 4T1. It can be seen from Fig. 5 that in the case of the angiostatin producing cells the infected mice developed smaller tumors, which were similar in size to the drug-treated tumor size.

### References

Boehm T, Folkman J, Browder T, O'Reilly MS. 1997 Antiangiogenic therapy of experimental cancer does not induce acquired drug resistance.Nature. 390(6658):404-7.
Cao Y, O'Reilly MS, Marshall B, Flynn E, Ji RW, Folkman J. 1998 Expression of angiostatin cDNA in a murine fibrosarcoma suppresses primary tumor growth and produces long-term dormancy of metastases. J. Clin. Invest. 101(5):1055-63.
Liu W., Ahmad S.A., Reinmuth N., Shaheen R.M., Jung Y.D., Fan F., Ellis L.M. 2000 Endothelial cell survival and apoptosis in the tumor. Apoptosis 5:323-328.
Moser T.L., Stack M.S., Wahl M.L, Pizzo S.V. 2002.The mechanism of action of angiostatin: Can you teach an old dog new tricks? ThrombHaemost 87:394-401. O'Reilly M.S., Holmgren L., Shing Y., Chen C., Rosenthal R.A., Moses M., Lane W.S., Cao Y., Sage E.H., Folkman J. 1994. Angiostatin: a novel angiogenesis inhibitor that mediates the suppression of metastases by a Lewis lung carcinoma. Cell 79:315-328.
Sim BK, O'Reilly MS, Liang H, Fortier AH, He W, Madsen JW, Lapcevich R, Nacy CA. 1997A recombinant human angiostatin protein inhibits experimental primary and metastatic cancer. Cancer Res. 57(7): 1329-34.
Wahl M.L., Moser T.L., Pizzo S.V. 2004. Angiostatin and anti-angiogenic therapy in human disease. Recent Prog Horm Res. 59:73-104.
Wahl M.L., Kenan D.J., Gonzalez-Gronow M., Pizzo S.V. 2005. Angiostatin's molecular mechanism: aspects of specificity and regulation elucidated. J Cell Biochem. 96:242-61.
Moser T.L., Stack M.S., Wahl M.L, Pizzo S.V. 2002.The mechanism of action of angiostatin: Can you teach an old dog new tricks? ThrombHaemost87:394-401.

## Claims

1. Chimeric fusion protein comprising angiostatin releasable therefrom by cleaving a protease-sensitive site comprised therein, wherein the fusion protein comprises a protease.

2. The fusion protein according to claim 1, wherein said protease-sensitive site is cleavable by the protease comprised in the fusion protein.

3. The fusion protein according to claim 1 or 2, wherein the amino acid sequences of angiostatin and the protease is separated by a linker sequence consisting of 3 to 15 amino acids, preferably comprising the protease sensitive site.

4. The fusion protein according to any of claims 1 to 3, wherein the protease is elastase.

5. The fusion protein according to any of claims 1 to 4, wherein the C terminus of the angiostatin is linked to the N terminus of the protease.

6. The fusion protein according to any of claims 1 to 5, wherein the N terminus of the angiostatin is linked to the C terminus of the protease.

7. The fusion protein according to any of claims 1 to 6, further comprising at least one targeting sequence, preferably for transporting the fusion protein to the extracellular space or to a specific type of cell or tissue.

8. Nucleic acid encoding the fusion protein according to any of claims 1 to 7.

9. Expression vector comprising the nucleic acid according to claim 8.

10. The expression vector according to claim 9, comprising regulatory sequences allowing constitutive or inducible expression.

11. Host cell comprising the expression vector according to claim 9 or 10.

12. The host cell according to claim 11, wherein the cell is a stem cell, most preferably mesenchymal stem cell, or other isolated cell, which can be grown to clinically effective cell numbers.

13. Chimeric fusion protein, comprising angiostatin releasable therefrom by cleaving a protease-sensitive site comprised therein, or a nucleic acid encoding said chimeric fusion protein, or a vector or host cell comprising said nucleic acid, for use in inhibiting angiogenesis, preferably in cancer or tumor.

14. Method for the inhibition of angiogenesis, comprising *in situ* release of angiostatin from the fusion protein according to claims 1 to 7, or from the protein encoded by the nucleic acid according to claim 8 or the expression vector according to claims 9 or 10, or from the host cell according to claims 11 to 12.

15. The method according to claim 14, wherein the angiostatin is released by autocatalysis, upon induction or constitutively.
